# EUROPEAN PATENT SPECIFICATION

(11) **EP 1 772 109 B1**
(45) Date of publication and mention of the grant of the patent: **22.04.2015**
(21) Application number: 05025424.2
(22) Date of filing: 22.11.2005
(51) Int. Cl.: A61B 18/14, A61B 5/046, A61B 5/042, A61B 17/00, A61B 18/00

(54) **System for performing cardiac ablation**
System für kardiale Ablationsbehandlungen
Dispositif d'ablation de tissu cardiaque

(30) Priority: 04.10.2005 US 243885
(43) Date of publication of application: 11.04.2007
(73) Proprietor: Covidien AG, 8212 Neuhausen am Rheinfall (CH)
(72) Inventor: Johnson, Kristin D., Louisville, CO 80027 (US); Ford-Serbu, Donna, Henderson, CO 80640 (US)
(74) Representative: Hoffmann Eitle

(56) References cited:
- WO-A-96/34571
- US-A- 5 233 515
- US-A- 5 246 438
- US-A- 5 643 197
- US-A- 6 132 426
- US-A1- 2004 199 161

## Description

### BACKGROUND

### 1. Field

The present disclosure relates generally to cardiac ablation surgical procedures, more particularly the present disclosure relates to an apparatus and method for ablating cardiac tissue to treat cardiac arrhythmias.

### 2. Description of the Related Art

Cardiac arrhythmia is a disturbance in the regular rhythm of the heart beat. A more serious variety of arrhythmia is known as atrial fibrillation (AF). This condition can be dangerous since it significantly reduces the heart's ability to properly circulate blood. AF is characterized by the chaotic quivering motion in the atria (i.e., the upper chambers of the heart). The quivering is caused by circular waves of electrical impulses that cyclically travel across the atria.

In AF, the sinoatrial node (i.e., the impulse generating tissue located in the right atrium of the heart) does not produce the regular impulses necessary for the rhythmic contraction of the heart. Instead, all tissue of the atrium discharges spontaneously, randomly generating an electrical impulse. More specifically, the locations where the electrical waves circulate have been identified to be in or around the pulmonary veins. This has allowed for the development of treatment techniques for AF which generally involve ablation of the tissue generating the irregular electrical impulses.

One of the more popular ablation methods involves the use of ablating electrodes which deliver radiofrequency (RF) energy to the target tissue thereby ablating the tissue and creating therapeutic lesions. A typical form of such ablation electrodes incorporates an insulated sheath from which an exposed (i.e., uninsulated) tip extends. Generally, the ablation electrode is coupled between a grounded RF power source (outside the body) and a reference electrode for contacting a large surface of the body, known as monopolar electrosurgery. When an RF voltage is provided between the reference electrode and the inserted ablation electrode, RF current flows from the ablation electrode through the body to the reference electrode. Typically, the current density is very high near the tip of the ablation electrode, which heats and destroys the adjacent tissue.

Another ablation technique may be based on bipolar electrosurgery, which involves placement of the ablation electrode (i.e., active electrode) and the reference electrode (i.e., return electrode) in proximity with each other. This arrangement contains the flow of RF energy to the target site. Usually, the two electrodes are arranged in a forcep-type instrument adapted to grasp tissue. As a result, such ablation instruments are more suitable for ablation of vessels, unlike monopolar ablation instruments, which are best suited for ablating tissue surfaces (e.g., organ walls).

During RF ablation, it is important to monitor the temperature that rises at the target tissue. Specifically, prior ablation electrodes should not exceed a predetermined temperature for at least two reasons. First, the temperature at the target site should not effectively exceed a temperature of 100°C, since at that temperature, the surrounding tissue will boil and char. Also, uncontrolled disruption, such as hemorrhage and explosive gas formation, may cause hazardous and clinically dangerous effects on the patient.

Second, maintaining proper temperature at the target site is essential because temperature directly relates to impedance, which affects the effectiveness and the extent of the therapeutic lesion. As temperature rises, the impedance rises as well, reducing the effectiveness of the lesion. However, conventional RF ablation electrodes used in treating AF are not capable of sensing and/or regulating the temperature at the target site to effectuate therapeutic lesions. Furthermore, these devices are also incapable of determining when the ablation is complete. Therefore, there is a need for an apparatus that can control the temperature at the target site during cardiac ablation as well as determine the completeness of the therapeutic lesion.

WO 96/34571 discloses an ablation electrode with a fluid coolant to a surface of the electrode for contacting tissue.

US 5,246,438 discloses a cardiac ablation apparatus having a distal monitoring electrode for picking up an endocardial potential.

US 5,233,515 discloses a real-time graphic display of heat lesioning parameters in a clinical lesion generator system.

US 2004/0199161 discloses an electrosurgical instrument having a tissue contacting surface of a PTC polymeric material. An active cooling system may be employed to cool the PTC material during use.

### SUMMARY

The present invention provides a system for performing cardiac ablation. The ablation system includes a generator supplying RF energy to an ablation electrode placed near or at the cardiac tissue requiring treatment. The ablation electrode includes one or more temperature control mechanisms, such as a positive temperature coefficient material or a coolant system, to regulate the temperature over the ablation electrode, thereby spreading the temperature over the surface of the electrode and increasing the reach of the ablation. In addition, the ablation system includes a cardiac sensor for recording electrical signals generated by the heart. The cardiac sensor is configured to record pre-treatment and post-treatment signals to determine when the treatment is complete and ablation should be terminated.

Accordingly, the invention provides an instrument for ablating tissue. The instrument-includes an electrode having a thermally-conductive tubular member with a closed distal end. The tubular member includes an external, electrically conductive outer surface adapted to connect to an electrical energy source with an insulation layer disposed thereon, thereby defining an exposed portion at the distal end. The instrument also includes one or more temperature control mechanisms to regulate temperature at the exposed portion and a cardiac sensor disposed within the interior cavity of the electrode configured to measure pre-treatment and post-treatment cardiac signals for comparison purposes. The instrument is configured to compare pre-treatment and post-treatment cardiac signals to determine the progress of tissue ablation and terminate the ablation based on the comparison.

There is also disclosed herein a method for performing cardiac ablation by creating an ablation lesion. The method includes the steps of placing an ablation electrode having one or more temperature control mechanisms in contact with a patient's heart. The ablation electrode includes a cardiac sensor disposed therein for measuring cardiac signals. The method also includes the steps of generating electrosurgical energy and supplying the electrosurgical energy to the patient through the ablation electrode. The method further includes the steps of regulating the temperature over the ablation electrode, thereby spreading the temperature over the surface of the electrode and increasing the volume of the ablation lesion, measuring and comparing pre-treatment and post-treatment cardiac signals to determine progress of tissue ablation, and terminating ablation based on the comparison of the pre-treatment and post-treatment cardiac signals.

Preferably, the electrode is coated with a positive temperature coefficient (PTC) material.

Preferably, the instrument also includes a fluid conduit within the tubular member which is connected to a source of selectively adjustable coolant supply for cooling tissue contiguous to the exposed portion. The instrument may further include a temperature sensor mounted proximate the electrically conductive outer surface configured to generate an output signal representative of a temperature proximate the electrically conductive outer surface. The coolant supply adaptively provides coolant according to the temperature at the electrically conductive outer surface.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 is a schematic diagram of an ablation electrode according to the present disclosure;
Fig. 2 is a block and sectional diagram of the ablation electrode of Fig. 1;
Fig. 3 is an ablation system according to the present disclosure;
Fig. 4 is an exemplary computing system for implementing the present disclosure;
Fig. 5 is a flow chart showing a method for controlling ablation; and
Fig. 6 is a graph showing temperature distributions for the ablation electrode of Fig. 1.

### DETAILED DESCRIPTION

Embodiments of the present disclosure will be described herein below with reference to the accompanying drawings. In the following description, well-known functions or constructions are not described in detail to avoid obscuring the present disclosure in unnecessary detail.

The foregoing disclosure describes a system and a method for performing cardiac ablation with reference to a monopolar ablation instrument. Those skilled in the art will understand that the present invention can be utilized in a bipolar ablation instrument.

The present disclosure provides for an ablation system including an ablation electrode having one or more temperature control mechanisms which regulate temperature over the ablation electrode, thereby spreading the temperature over the surface of the electrode and increasing lesion volume. The temperature control mechanisms may be any of the following, alone or in combination, a positive temperature coefficient coating the ablation electrode and a coolant system. In addition, the ablation electrode includes a cardiac sensor for measuring signals generated by the heart to determine when ablation is complete.

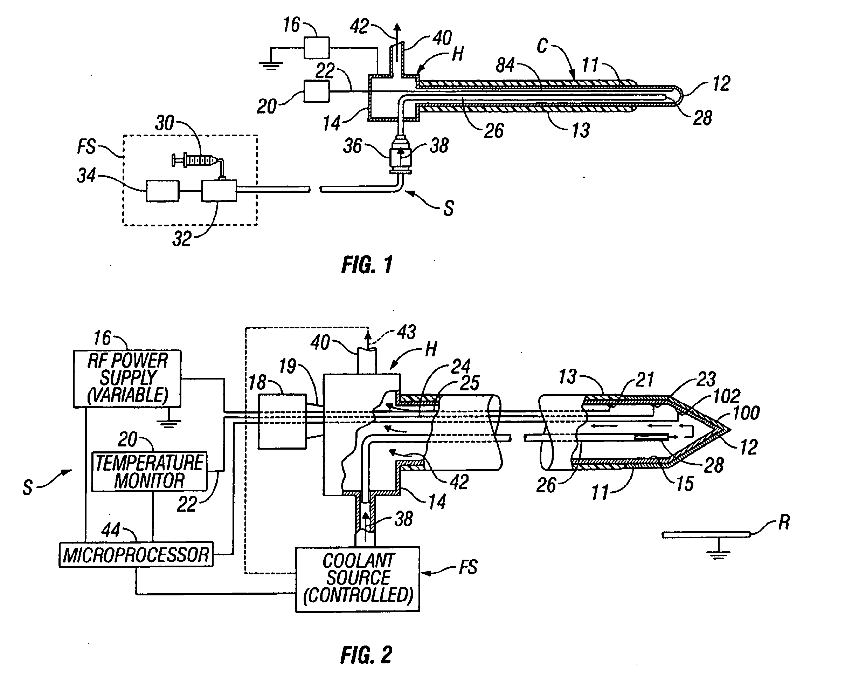

Referring to Figs. 1 and 2, an ablation system is shown, which incorporates an elongated shaft or cannula body C configured for insertion, either percutaneously or intraoperatively into an open wound site at the target site in or around the heart. As illustrated the cannula body C is integral with a head or hub element H coupled to remotely support components, collectively designated S.

As shown in Figs. 1 and 2, the cannula body C incorporates an elongated hollow ablative electrode 11 formed of conductive material, (e.g. metal such as stainless steel, titanium, etc.). At the distal end of the cannula body C, the electrode 11 includes a shaft 15 which defines a tip 12 at a distal end thereof which may be of any shape or form (e.g., radiused or pointed). In one form, the tip 12 may define a trocar point and may be of robust metal construction to facilitate insertion or penetration of tissue. During an ablation procedure, an RF power supply 16 provides electrical current which spreads from the conductive portion, e.g., tip 12 to pass through the surrounding tissue thereby ablating the tissue and creating therapeutic lesions. Hence, when the tip 12 is positioned contiguous to tissue, energy from the RF power supply 16 is dissipated into heat within the tissue.

As best shown in Fig. 2, the electrode 11 includes an insulative coating 13 for preventing the flow of electrical current from the shaft 15 of electrode 11 into surrounding tissue. Thus, the insulative coating 13 shields the intervening tissue from RF current, so that such tissue is not substantially heated along the length of the shaft 15 except by the heating effect from the exposed portion of tip 12. It should be appreciated that the length of the exposed portion of tip 12 is directly related to the size of the lesion created (i.e., the larger the exposed portion of the electrode 11 the larger the lesion).

At its proximal end, the electrode 11 is typically integrally associated with an enlarged housing 14 of the hub H which carries electrical and coolant connections as explained in greater detail below. Outside the patient's body, the housing 14 defines ports for connections to the support components S (e.g., electrical and fluid couplings). As suggested, the housing 14 may be integral with the electrode 11, formed of metal, or it may constitute a separate subassembly as described below. Alternatively, the housing 14 can be made of plastic, accommodating separate electrical connections. In that regard, a plastic housing 14 is preferred, due to low artifact imaging it exhibits in various imaging techniques (e.g., X-ray, CT, MRI, etc.) as is known in the art.

Referring to Fig. 2, the housing 14 mates with a block 18 thereby defining a luer taper lock 19 which seals the block 18 and the housing 14. In addition, fluid and electrical couplings are provided. Specifically, connection to a regulated RF supply 16 (e.g., the cables 3, 5 of Fig. 1) may be a standard cable connector, a leader wire, a jack-type contact or other connector designs known in the art. The temperature-sensing and radiofrequency electrical connections can be made through the housing 14 and extend to the region of the tip 12, where an RF line 25 is connected by junction 21 (e.g., a weld, braze, or other secure electrical connection). Sensor lines 24 extends to a temperature sensor 23 (e.g., a thermistor, a thermocouple, or other type of sensor) which may be fused or in thermal contact with the wall of the tip 12 to sense temperature condition at or proximate tip 12.

The RF power supply 16 may be connected to reference potential and coupled through the block 18 affixed to the hub H. Specifically, the RF power supply 16 provides RF voltage through the block 18 with an electrical connection to the electrode 11 as indicated by the line 25, to the connection junction 21.

During ablation, the electrical circuit is completed through the body using a reference or dispersive electrode R that is connected elsewhere to the body. The RF energy passes from the RF power supply through the ablation electrode 11 and the patient's body to the electrode R. Consequently the RF power supply 16 heats body tissue by current from the tip 12.

The RF power supply 16 may be connected to reference potential and coupled through the block 18 affixed to the hub H. Specifically, the RF power supply 16 provides RF voltage through the block 18 with an electrical connection to the electrode 11 as indicated by the line 25 (e.g., the cables 3, 5), to the connection junction 21. The RF power supply 16 may take the form of an RF generator as exemplified by the RFG-3C RF Lesion Generator System available from Radionics, Inc. of Burlington, Massachusetts.

The ablation electrode 11 includes a number of systems for regulating the temperature generated at the ablation site. One such system utilizes cooling fluid injected into the ablation electrode 11 based on temperature readings. In that regard, a temperature monitor 20 is electrically connected by lines 22 and 24 to the temperature sensor 23 as in the form of a thermocouple or thermistor typically within or contacting the tip 12. As illustrated, the temperature sensor 23 is connected to the tip 12. The sensed temperature is utilized to control either or both of the flow of RF energy or the flow of coolant to attain the desired ablation while maintaining the maximum temperature substantially below 100°C or another threshold temperature. A plurality of sensors may be utilized including units extending outside the tip 12 to measure temperatures existing at various locations in the proximity of the tip 12. The temperature monitor 20 may be as exemplified by the TC thermocouple temperature monitoring devices available from Radionics, Inc. of Burlington, Massachusetts.

Temperatures at, or near the tip 12 may be controlled by adjusting the flow of fluid coolant through the ablation electrode 11. Accordingly, the temperature of the tissue contacting at or near the tip 12 is controlled. In one disclosed embodiment, fluid from a fluid source FS is carried the length of the ablation electrode 11 through a tube 26 extending from the housing H to the distal end of the electrode 11 terminating in an open end 28 at the tip 12. At the opposite end of the electrode 11, within the housing H, the tube 26 is connected to receive fluid. As illustrated in the detailed structure of Figs. 1 and 2, the fluid source FS includes a source unit 34 coupled through a control 32 utilizing a hypodermic syringe 30 (or other fluid delivery mechanism) to actuate fluid flow, as represented by an arrow, through a coupling 38. Thus, fluid flow is regulated in accordance with observed temperature, allowing increased flow of RF energy.

The fluid coolant may take the form of water or saline solution which is typically used for heat dissipation from the tip 12. The reservoir or source unit 34 might be a large reservoir of coolant fluid. As a simplistic example, a tank of water with ice cubes can function to maintain the coolant at a temperature of approximately 0 °C. As another example, the fluid source FS could incorporate a peristaltic pump or other fluid pump, or could merely be a gravity feed for supplying fluid from a flexible bag or rigid tank.

Backflow from the tip 12 is through an exit port 40 of the hub H as illustrated by arrows 42 and 43. The port 40 may be in the form of simple couplings, rigid units or may comprise flexible tubular couplings to reduce torque transmission to the electrode 11. Also, the coolant flow members may simply take the form ofPVC tubes with plastic luer connectors for ease of use.

As a result of the coolant flow, the interior of the electrode 11, more specifically the electrode tip 12, can be held to a temperature near that of the fluid source FS. The coolant can circulate in a closed system as illustrated in Fig. 2. Also, in some situations, it may be desirable to reverse the direction of fluid flow from that depicted in the figures. As treated in detail below, coordinated operation, involving RF heating along with the cooling may be accomplished by a microprocessor 44, which is coupled to the RF power supply 16, the temperature monitor 20 and the fluid source FS to receive data on flow rates and temperatures and exercise control. Accordingly, an integrated operation is provided with feedback from the temperature monitor 20 in a controlled format and various functions can be concurrently accomplished. Thus, facilitated by the cooling, the ablation electrode 11 is moderated, changed, controlled or stabilized. Such controlled operation can effectively reduce the temperature of tissue near the tip 12 to accomplish an equilibrium temperature distribution tailored to the desired size of the desired lesion.

The temperature distribution in the tissue near the tip 12 depends on the RF current from the tip 12 and depends on the temperature of the tissue which is adjacent to the tip 12. Tip temperature can be controlled by the flow of fluid from the source FS. Thus, a thermal boundary condition is established, holding the temperature of the tissue (near the tip 12) to approximately the temperature of the tip itself, e.g. the temperature of the fluid inside the tip 12. Accordingly, by temperature control, a surgeon may impose a defined temperature at the boundary of the electrode tip 12 which can be somewhat independent of the RF heating process, and in fact, dramatically modify the temperature distribution in the tissue.

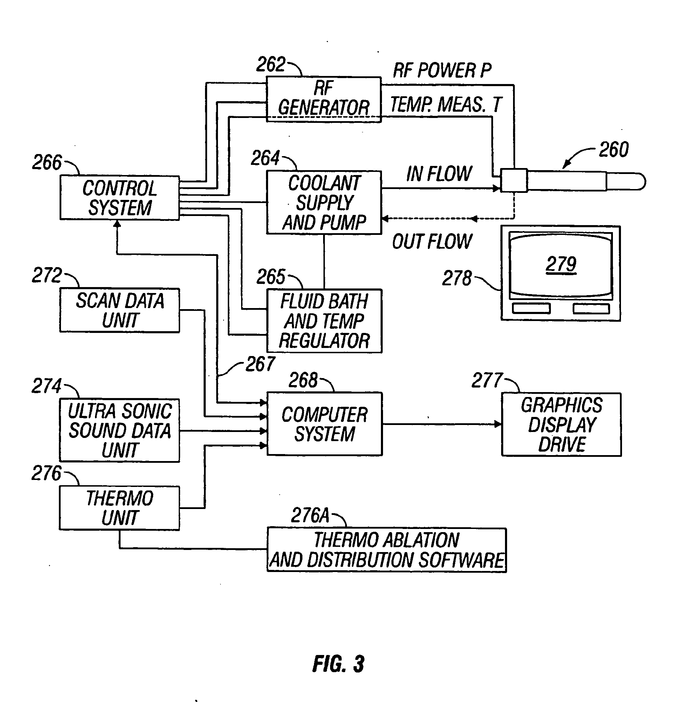

The control mechanisms of the coolant system will now be discussed. Fig. 3 shows a control system for an ablation electrode structure 260 which may take any of multiple forms including the embodiments described above (i.e., the ablation electrode 11). The electrode structure 260 is energized by an RF generator 262 and cooled by coolant supplied from a source 264. A control system 266 regulates various parameters (e.g., RF energy output, coolant flow, etc.) in accordance with a predetermined program stored within a computer system 268. Note that various forms of feedback control systems are well known and may be implemented in the computer system 268.

Functionally, the computer system 268 receives feedback parameters through a bus 267 from the control system 266 which in turn, executes the desired program. The parameters are processed through a monitoring and feedback program implemented within the computer system 268. A simple two-parameter control system can be implemented by the control system 266 in conjunction with the computer system 268 and input data from a scan data unit 272 and an ultrasonic sound data unit 274 involving a thermal distribution calculation by the computer system 268 as illustrated. Thus, the computer system 268 also receives data from a plurality of sources, specifically the scan data unit 272, the sound data unit 274 and a remote temperature unit 276 operating with ablation and distribution software 276A. Accordingly, in addition to implementing a basic ablation control program, the computer system 268 provides raw display data to a graphics display drive 277 for actuating a display unit 278. Thus, multiple displays are available on a screen 279, for example, slicings, time courses, reformattings, and digital subtraction representations, as well as digital and analog meter representations.

The scan data unit 272 stores two or three dimensional graphics data relating to the surgery target to be provided selectively so that a surgeon may visualize the anatomy prior to, during and after the procedure. The data stored by the scan unit 272 may take the form of CT or MRI data developed prior to the surgical event as well known. The data may be either stereotactic or non-stereotactic involving immobilizers, fiducial marks, graphic reference mechanisms and so on.

The sonic data unit 274 may take a form well known in the art to provide sonic data, as from a stethoscope, electronic microphone or sonic detector to visualize tissue. For example, the data is provided and processed to display the electrode structure 260 with respect to anatomy. In that regard, signal represented data from the sonic data unit 274 and the scan data unit 272 may be combined by the computer system 268 to provide display signals for composite displays. Various other displays may be provided to inform and guide the procedure as it is somewhat controlled with respect to the flows of energy and coolant. In that regard, the program may be implemented to include calculation algorithms, look-up tables, heuristic algorithms, historical clinical data, mathematical calculations involving field and thermal distribution calculations by finite element methods, analytical form solutions, computer theoretic methods, any or all of which may be used to analyze and process image data as well as operation procedures.

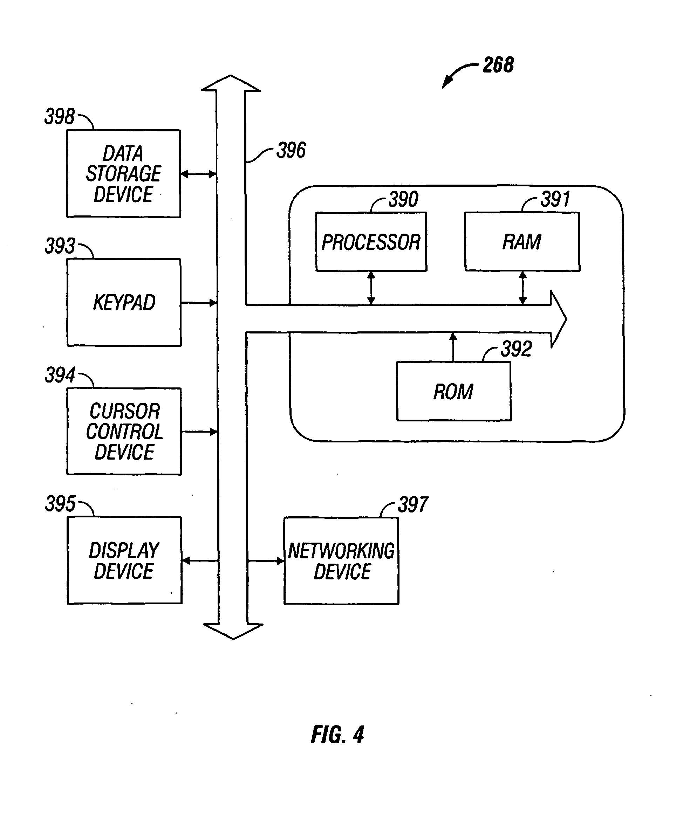

The components of the computer system 268 are shown in Fig. 4. It is to be understood that the present disclosure may be implemented in various forms of hardware, software, firmware, special purpose processors, or a combination thereof. In one embodiment, the present disclosure may be implemented in software or firmware as an application program tangibly embodied on the computer system 268.

The computer system 268 may include one or more central processing units (CPU) 390, a random access memory (RAM) 391, a read only memory (ROM) 392 and input/output (I/O) interface(s) such as a keypad 393, cursor control device 394 (e.g., a mouse, touchscreen, etc.), a data storage device 398, and display device 395. Furthermore, the computer system 268 may also include a networking device 397 which provides wired or wireless connectivity to the network 16. In addition, various other peripheral devices may be connected to the computer system 268 by various interfaces and bus structures, such as a parallel port, serial port or universal serial bus (USB). A system bus 396 couples the various components and may be any of several types of bus structures including a memory bus or memory controller, a peripheral bus, and a local bus using any of a variety of bus architectures.

The computer system 268 also includes an operating system and micro instruction code. The various processes and functions described herein may either be part of the micro instruction code, firmware, or part of the application program (or a combination thereof) which is executed via the operating system. In addition, the computer system 268 includes software for displaying user input screens and recording user responses.

It is to be further understood that because some of the constituent system components and method steps depicted in the accompanying figures may be implemented in software, the actual connections between the system components (or the process steps) may differ depending upon the manner in which the present disclosure is programmed. Given the teachings of the present disclosure provided herein, one of ordinary skill in the related art will be able to contemplate these and similar implementations or configurations of the present disclosure.

A look-up table or function generator defines the ablation volume as a function of the tip geometry and tip temperature. The tip temperature, To, could be clamped at a fixed value by cooling fluid or if uncooled, the value T₀ is measured by temperature sensors. Using tables such as described in the paper of Cosman, et al., entitled "Theoretical Aspects of Radiofrequency Lesions in the Dorsal Root Entry Zone," Neurosurgery 15, 945-950, 1984, one could predict the width or minor diameter of the prolate ellipsoid of revolution which represents the ablation isotherm and corresponding to say a given power output level from the lesion generator at a given tip temperature near the electrode. This could either be derived empirically from experimental data or could be calculated from the equilibrium equation where K is the tissue thermal conductivity, σ is the tissue electrical conductivity, T is the temperature in the tissue, and dQ_{c} /dt is the rate of heat loss due to blood circulation, as discussed in Cosman, et al. Therefore, the surface of revolution corresponding to the ablation temperature of approximately 50°C could be determined as a functional equation, S(T₀,R₀,L₀,P₀,x,y,x)=0.

This equation represents the surface of revolution using the x,y,z coordinates relative to the tip of the electrode as a function of the tip radius parameter R₀, tip length L₀, the tip temperature To, and the power P₀ of the RF lesion generator. This surface S could be displayed in the coordinate system of the electrode or in the 3D coordinate system of the CT or MR data or in a stereotactic coordinate system space referenced to a localizer structure, or localizer marker(s), or external apparatus (arc, frame, etc.) near the patient. The surface could be displayed on the computer as a red spheroid around the tip. Its relation to the defined lesion volume could be obvious by graphic rendering such as done for radiosurgery in the XKnife product of Radionics, Inc. of Burlington, Massachusetts.

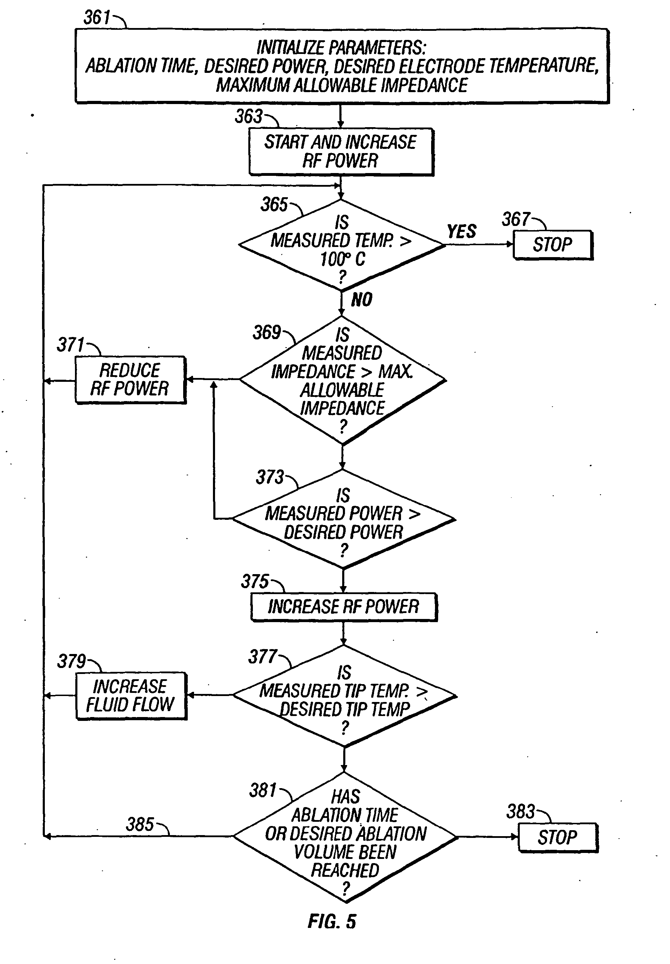

A method for implementation by the computer system 268 is illustrated in Fig. 5. In step 361, an initializing operation of setting parameters occurs. More specifically, ablation time, power, electrode temperature, and allowable impedance are all initialized. Thereafter, the process is initiated with the established parameters as indicated by the step 363. From that stage, the data is monitored. Specifically, the temperature is measured as indicated in the various disclosed embodiments. As indicated by the query step 365, if a temperature in excess of 100 °C is measured, the procedure is terminated in step 367.

If temperatures are below the critical level, the maximum allowable impedance is determined. That is, as indicated by the query step 369, if the temperature is exceeded, the RF power is reduced in step 371. In that regard, note that temperature is indicated to be checked by the program at predetermined intervals. In fact, the system may maintain a continual observation of temperature with an override to terminate the procedure at any time if excessive values are observed. However, for illustrative purposes, the program is described in a step process.

Acceptable levels of temperature and impedance are established in steps 365 and 369 respectively and the power is measured with respect to the desired level in step 373. An excessive level results in a power reduction in step 371, otherwise, if power is low, it is increased in step 375. Thus, power is adjusted to attain the desired level.

With the desired level of power established, the tip temperature is measured in step 377. An excessive level of tip temperature actuates an increase in the flow of coolant in step 379 and a check of the other parameters. Otherwise, the final query is made in step 377, specifically, whether the desired ablation volume (e.g., volume of the therapeutic lesion) has been attained. If so, the procedure is terminated in step 383, otherwise, as indicated by the directional process flow line 385, the operation is repeated, returning to the step 365.

In addition, to the coolant system disclosed above, the ablation electrode 11 may include, either in combination or alone, another temperature control mechanism. This system utilizes a positive temperature coefficient (PTC) material on, or preferably, coating the ablation electrode 11, more specifically the tip 12. PTC materials respond to increases in localized temperature by increasing local resistance which in turn reduces current flow and lowers the temperature. This characteristic is utilized in the present disclosure to regulate the heat generated at the ablation site by increasing the resistance which decreases the RF energy passing through ablation electrode 11.

Heat is generated in the following manner during ablation. The area of the ablation electrode 11 that is in contact with the ablation site (i.e., the tip 12) affects the current density of the signal that heats the tissue. The smaller the contact area the ablation electrode 11 has with the tissue, the greater the current density and the greater and more concentrated the heating of tissue is. Conversely, the greater the contact area of the ablation electrode 11, the smaller the current density and the less heating of the tissue. Further, the greater the heating of the tissue, the greater the probability of burning the tissue. It is therefore important to either ensure a relative high amount of contact area between the ablation electrode 11 and the tissue, or otherwise maintain a relatively low current density on the ablation electrode 11.

While there are various methods of maintaining a relatively low current density (including, inter alia, the use of electrosurgical return electrode monitors (REMs), such as the one described in commonly-owned Patent No. 6,565,559, the present disclosure ensures the ablation electrode 11 maintains a low current density by distributing the temperature created by the current over the surface of the ablation electrode 11.

According to another embodiment of the present disclosure, current density at the ablation electrode 11 is reduced via a PTC layer 100 disposed on the ablation electrode 11. As best illustrated in Fig. 2, ablation electrode 11 is coated with a PTC layer 100. The PTC layer 100 can be made of, for example, polymer/carbon based material, a cermet based material, a polymer material, a ceramic material (e.g., barium titanate), a dielectric material, or any combinations thereof. An example of such material that can be used for the PTC material is described in U.S. Patent No. 6,132,426, and is known as "PolySwitch®" made by the Raychem Corporation of California.

The PTC layer 100 acts to distribute the temperature created by the current over the surface of the ablation electrode 11 to minimize the risk of patient burns. The PTC layer 100 regulates the temperature over the area of the ablation electrode 11 by responding to increases in temperature with an increase in resistance in localized areas. The increase in resistance reduces the current in the localized area, thus causing the current to conduct more in the areas with lower resistance or lower temperature. Further, as current is applied through the PTC layer 100 of ablation electrode 11, power is dissipated and the temperature is increased. The increase in temperature increases the resistance and limits the current, thus reducing the heating effect. This equalizes the temperature throughout the entire surface of the tip 12. Consequently, there are no localized "hot spots," which typically cause patient bums. As the overall temperature increases, consequently increasing the resistance, an REM (return electrode monitoring) circuit can detect and measure such increases and turn off an RF supply when a predetermined temperature has been exceeded.

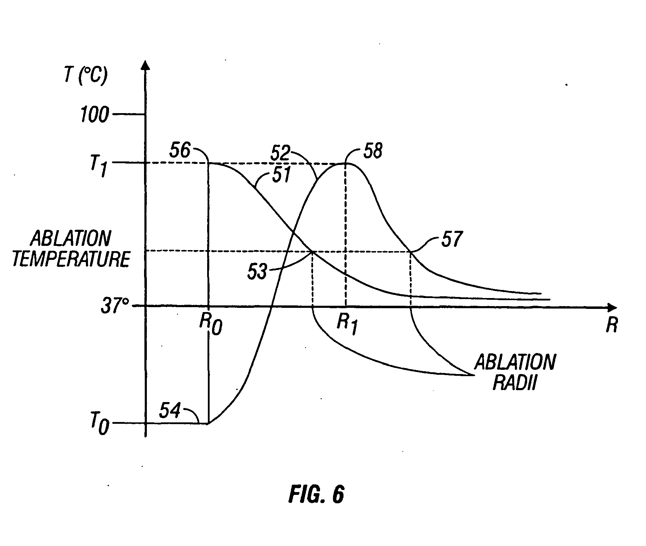

To consider the effect of temperature distributions from the tip 12, reference now will be made to the graph of Fig. 6, which shows the benefits of decreasing temperature at the ablation site. The nominal radial distance R from the central axis of an electrode tip is plotted against temperature T. In the illustrated example, a nominal radius R₀, representing the surface of the ablation electrode 11, is depicted. A body temperature of 37°C is the base reference line in the graph. Also, a temperature level of 100°C is indicated; the boiling point of water and essentially that of body tissue. As explained above, such a temperature is highly undesirable in any controlled clinical setting. Accordingly, it is important to maintain the temperature of the electrode substantially below 100°C.

The curve 51 represents the operation of a traditional ablation electrode, whereby at the electrode surface (i.e., R₀) the tissue is elevated to a safe temperature T₁. However, from that point the temperature rapidly falls off and approaches body temperature 37°C asymptotically as the distance R increases from the electrode.

It is generally accepted that most bodily tissue across most cell lines will permanently die if held at a temperature in the range of 45°C to 60°C for a sustained period, e.g. 60 seconds. Accordingly, the ablation radius for a lesion generally corresponds to the radius associated with temperatures in a range of 45°C to 60°C. Thus, ablation by the electrode as depicted by the curve 51 would be effective only to the radius of a point 53.

The curve 52 illustrates the characteristic of an electrode or ablation system in accordance with the present invention. The ablation electrode 11 is maintained at an approximate temperature, e.g. temperature T₀, as indicated, a substantially lower temperature than the body temperature of 37°C. A substantially horizontal section 54 of the curve 52 indicates that the ablation electrode 11 is held at a constant temperature T₀ within the radius R₀. The section 54 represents a situation in which the interior of the ablation electrode 11 is held at a temperature T₀ by circulating coolant. Such operation imposes the boundary condition at R₀ such that the tissue outside the tip is also substantially at the temperature T₀.

Considering further representations of the curve 52, the RF current causes energy dissipation in the tissue immediately adjacent to and distanced from the electrode radius R₀, but the equilibrium temperature distribution of the tissue is controlled by the equation of heat disposition, conduction and convection throughout the space. Since the ablation electrode 11 is held at the temperature T₀, the temperature curve 52 must be continuous and must meet the point T₀ at radius R₀. As a result, the heating causes higher temperatures at greater distances from the tip as shown by the rise of the curve 52 to a maximum temperature T₁ at a radius R₁ substantially greater than the radius R₀. The actual ablation radius is indicated at a point 57, substantially displaced from the point 53.

Beyond the radius R₁, blood convection dominates to a larger radius and as illustrated, the curve 52 falls off to its asymptotic limit approximating 37°C. The curve 52 illustrates that by cooling in the improved electrode tip, the radius R₁ corresponding to a temperature T₁ is much larger than the radius corresponding to the same temperature T₁ for traditional electrodes. Thus, by cooling the electrode tip, the zone of highest temperature is extended, since the radius is increased, (e.g., R₁), further away from the ablation electrode 11 than the radius R₀ of traditional electrodes; similarly the ablation radii as indicated by the points 53 and 57.

The consequence of lowering the temperature at the ablation site is a larger radius of the ablation lesion. Hence, the ablation radius can be made substantially larger for an ablation electrode equipped with temperature regulatory components discussed above, than for a convention electrode of essentially identical shape and form without similar technologies. This is illustrated by the radius of the lesion represented by the point 57 on the curve 52 compared to the point 53 on the curve 51. Implementations in accordance with the disclosed embodiments in actual living tissue, indicate that with an electrode of 20 gauge (a radius of under 1 mm) lesion sizes can be expanded from a limited range of approximately 10 mm in diameter to diameters of 20 to 30 mm.

In a clinical setting, systems according to the present disclosure offer a significant advantage over conventional ablation electrodes since they allow for creation of larger therapeutic lesions in fewer passes. Conversely, with traditional electrodes, multiple passes or multiple off-access electrode passes would be required with all of the incumbent disadvantages and hazards of hemorrhage, discomfort, risk of hitting critical structures, heterogeneities of temperature distributions, and the risk of not ablating the entire volume of concern.

In addition to including temperature sensing and controlling capabilities, the ablation system according to the present disclosure also includes sensing equipment for measuring electrical cardiac signals. The sensing equipment measures cardiac signals which are used to determine whether the ablation procedure has finished treating atrial fibrillation (AF). Conditions, such as AF, affect the electrical signals generated by the heart, therefore electrical signals of a heart affected by AF are different from those of a healthy heart. Comparing the two types of signals allows for determination whether the ablation is complete and should be stopped.

The ablation electrode 11 shown in Fig. 2 includes a cardiac signal sensor 102 which records electrical signals (e.g., ECG signals) generated by the heart and transmits them to the microprocessor 44. The cardiac sensor 102 records signals throughout the ablation procedure. The microprocessor 44 processes the received signals to generate an electrocardiogram (ECG) which may be outputted either on a monitor or a printer. Once the therapeutic lesion has been created, the heart is cured of AF. As a result the heart will generate electrical signal indicative of a healthy condition. Since, an ECG of a heart afflicted by AF and post-ablation ECG are different, the data the signals provide is used to determine that the ablation procedure is complete. The decision to terminate the ablation may be made by the surgeon if the ECG is being outputted by the microprocessor 44. In addition, the microprocessor 44 may analyze the signals either in addition to or without providing an output for the surgeon using the ablation electrode 11. The microprocessor 44 compares the pre-treatment ECG and the post-treatment ECG to determine whether the ablation is complete. Once the microprocessor 44 determines that the ablation is complete it may shut down the RF power supply 16 automatically or send a signal to generate a stoppage alarm (e.g., audio and/or visual) indicating that the ablation is complete. It is also envisioned that pulses may be used to measure completeness during off times (e.g., when energy is not being supplied to the electrode 11).

The above embodiment discloses the cardiac sensor 102 incorporated into the ablation electrode 11. The above embodiment is one preferred configuration, since it places the cardiac sensor 102 within the tip 12 which allows for monitoring of cardiac signals at the ablation site. This allows for more accurate measurements of the cardiac signals. The cardiac sensor is disposed within the electrode according to claim 1. However, it is disclosed that those skilled in the art will appreciate that a cardiac sensor may be a stand alone device, which is not embedded in the ablation electrode 11 and can be placed in or around other segments of the patient's body.

It is also envisioned that the energy delivery to the electrode 11 may be controlled based on measured impedance at the target tissue. As the impedance of the tissue changes the current changes inversely to the impedance, if the voltage remains constant. This is defined by Ohm's law: V = RI, wherein V is the voltage across the electrodes in volts, I is the current through the electrodes (and tissue) in milliamps and R is the resistance or impedance of the tissue measured in Ohms. By this equation it can be readily appreciated that when the tissue impedance increases, the current will decrease and conversely, if the tissue impedance decreases, the current will increase. The electrosurgical system of the present disclosure essentially measures impedance based on the changes in current. Prior to electrosurgical treatment, tissue is more conductive, so when energy is applied, the impedance is relatively low. As the tissue is treated and a lesion is created, the conductivity decreases as the tissue moisture content decreases and consequently tissue impedance increases.

RF power supply 16 includes a current sensor (not shown) electrically connected to the electrode 11 and a voltage sensor (not shown) electrically connected between the electrode 11 and return electrode R. The current sensor measures the current and the voltage sensor detects the voltage between at the target tissue. The current and voltage sensors feed analog voltage and current signals to analog to digital converters (not shown).

The analog to digital converters receive the analog signals and convert it to a digital signal for transmission to the microprocessor 44, which may include a comparator and a controller. An output port of the microprocessor 44 is electrically connected to the RF power supply 16. The microprocessor 44 calculates the impedance according to by Ohm's law.

The comparator evaluates the digital impedance signal by comparing it to predetermined impedance values and generates responsive signals for transmission to the controller as described in detail below. In response to the signals received from the comparator, the controller generates and transmits control signals to the RF power supply 16 which in turn controls the energy output to the electrode 11. The control signal may include a command to adjust the RF power supply 16 to supply energy to maintain a predetermined impedance value.

It is further envisioned that the temperature control mechanisms of the present disclosure can be applied to bipolar electrosurgical systems (e.g., electrosurgical forceps). For example, forceps typically include a pair of opposable jaw members when in closed position are configured to grasp tissue. The jaw members include electrosurgical sealing plates having temperature control mechanisms disclosed above. Examples of bipolar electrosurgical forceps are shown and described in commonly-owned U.S. Publication No. 2004/0182645 entitled "VESSEL SEALER AND DIVIDER AND METHOD MANUFACTURING SAME" and U.S. Patent Publication No. 2005/0021027 entitled "TISSUE SEALER WITH NON-CONDUCTIVE VARIABLE STOP MEMBERS AND METHOD OF SEALING TISSUE". It is also envisioned that the sealing plates can be configured to produce narrow band of sealing with controlled energy delivery if the plates are offset a predetermined distance.

The described embodiments of the present disclosure are intended to be illustrative rather than restrictive, and are not intended to represent every embodiment of the present disclosure. Various modifications and variations can be made without departing from the scope of the disclosure as set forth in the following claims.

## Claims

1. An instrument for ablating tissue, comprising:
an electrode (11) including a thermally-conductive tubular member (15) with a closed distal end (12), the tubular member defining an external, electrically conductive outer surface adapted to connect to an electrical energy source, the electrode having an insulation layer (13) disposed on the external, electrically conductive outer surface and an exposed portion at the distal end;
at least one temperature control mechanism to regulate temperature at the exposed portion of the electrically conductive outer surface; **characterised by**:
a cardiac sensor (102) disposed within the interior cavity of the electrode configured to measure pre-treatment and post-treatment cardiac signals for comparison purposes;
the apparatus further comprising a microprocessor (44) that is configured to:
compare the pre-treatment and post-treatment cardiac signals to determine progress of tissue ablation; and
terminate ablation based on the comparison of the pre-treatment and post-treatment cardiac signals.

2. An instrument according to claim 1, wherein the cardiac sensor is disposed adjacent the distal end of the electrically conductive outer surface.

3. An instrument according to claim 1 or 2, wherein the at least one temperature control mechanism comprises:
a fluid conduit (26) defined within the tubular member and adapted to be connected to a source (FS) of selectively adjustable coolant supply for cooling tissue contiguous to the exposed portion of the electrically conductive outer surface.

4. An instrument according to claim 3, wherein the at least one temperature control mechanism further comprises:
a temperature sensor (23) mounted proximate the electrically conductive outer surface configured to generate an output signal representative of a temperature proximate the electrically conductive outer surface, the selectively adjustable coolant supply adaptively providing coolant according to the temperature proximate the electrically conductive outer surface.

5. An instrument according to claim 4, wherein the selectively adjustable coolant supply is configured to automatically maintain the tissue contiguous to the electrically conductive outer surface at a temperature below 100°C.

6. An instrument according to claim 4 or 5, wherein the selectively adjustable coolant supply is configured to automatically maintain the tissue contiguous to the electrically conductive outer surface at a temperature above 37 °C.

7. An instrument according to any one of the preceding claims, wherein the at least one temperature control mechanism comprises a positive temperature coefficient (PTC) material (100) coating on the electrode.

8. An instrument according to claim 7, wherein the PTC material is selected from the group consisting of a polymer/carbon based material, a cermet based material, a polymer material, a ceramic material, a dielectric material, and any combinations thereof.

9. An instrument according to claim 8, wherein the PTC material is a polymer/carbon based material.

10. An instrument according to claim 8, wherein the PTC material is a cermet based material.

11. An instrument according to claim 8, wherein the PTC material is a polymer material.

12. An instrument according to claim 8, wherein the PTC material is a ceramic material.

13. An instrument according to claim 8, wherein the PTC material is a dielectric material.

14. An instrument according to any one of claims 7 to 13, wherein the PTC material maintains the tissue contiguous to the electrically conductive outer surface at a temperature below 100°C.

15. An instrument according to any one of claims 7 to 14, wherein the PTC material maintains the tissue contiguous to the electrically conductive outer surface at a temperature above 37°C.

16. The instrument according to any one of claims 1 to 15, configured to terminate ablation based on the comparison of the pre-treatment and post-treatment cardiac signals by shutting down the electrical energy source.

17. The instrument according to any one of claims 1 to 15, wherein the cardiac sensor is disposed within the exposed portion at the distal end of the electrode.

## Patentansprüche

1. Instrument für Gewebeablation, mit:
einer Elektrode (11) einschließlich eines thermisch leitfähigen rohrförmigen Elements (15) mit einem geschlossenen distalen Ende (12), wobei das rohrförmige Element eine außenliegende, elektrisch leitfähige Außenfläche definiert, die eingerichtet ist, sich mit einer elektrischen Energiequelle zu verbinden, wobei die Elektrode eine Isolationsschicht (13), die auf der außenliegenden, elektrisch leitfähigen Außenfläche angeordnet ist, und einen exponierten Abschnitt bei dem distalen Ende aufweist;
zumindest einem Temperatursteuermechanismus, um die Temperatur bei dem exponierten Abschnitt der elektrisch leitfähigen Außenfläche zu regeln; **gekennzeichnet durch**:
einen Herzsensor (102), der in dem inneren Hohlraum der Elektrode angeordnet ist, die eingerichtet ist, für Vergleichszwecke Herzsignale vor und nach einer Behandlung zu messen;
die Vorrichtung ferner mit einem Mikroprozessor (44), der eingerichtet ist:
die Herzsignale vor und nach der Behandlung zu vergleichen, um den Fortschritt der Gewebeablation zu bestimmen; und
die Ablation basierend auf dem Vergleich der Herzsignale vor und nach der Behandlung zu beenden.

2. Instrument nach Anspruch 1, bei dem der Herzsensor angrenzend an das distale Ende der elektrisch leitfähigen Außenfläche angeordnet ist.

3. Instrument nach Anspruch 1 oder 2, bei dem der mindestens eine Temperatursteuermechanismus aufweist:
einen Fluidkanal (26), der in dem rohrförmigen Element definiert und eingerichtet ist, mit einer Quelle (FS) gezielt einstellbarer Kühlmittelzufuhr zum Kühlen von Gewebe verbunden zu sein, das an den exponierten Abschnitt der elektrisch leitfähigen Außenfläche angrenzt.

4. Instrument nach Anspruch 3, bei dem der mindestens eine Temperatursteuermechanismus ferner aufweist:
einen Temperatursensor (23), der nahe der elektrisch leitfähigen Außenfläche montiert ist und eingerichtet ist, ein Ausgangssignal zu erzeugen, das eine Temperatur nahe der elektrisch leitfähigen Außenfläche wiedergibt, wobei die gezielt einstellbare Kühlmittelzufuhr adaptiv Kühlmittel entsprechend der Temperatur nahe der elektrisch leitfähigen Außenfläche bereitstellt.

5. Instrument nach Anspruch 4, bei dem die gezielt einstellbare Kühlmittelzufuhr eingerichtet ist, das Gewebe an der elektrisch leitfähigen Außenfläche automatisch bei einer Temperatur von unter 100 °C zu halten.

6. Instrument nach Anspruch 4 oder 5, bei dem die gezielt einstellbare Kühlmittelzufuhr eingerichtet ist, das an der elektrisch leitfähigen Außenfläche angrenzende Gewebe bei einer Temperatur von über 37 °C zu halten.

7. Instrument nach einem der vorstehenden Ansprüche, bei dem der mindestens eine Temperatursteuermechanismus ein Material (100) mit einem positiven Temperaturkoeffizienten (PTC) aufweist, das die Elektrode bedeckt.

8. Instrument nach Anspruch 7, bei dem das PTC-Material aus der Gruppe ausgewählt ist, die aus einem polymer-/kohlenstoffbasierten Material, einem Cermet-basierten Material, einem Polymermaterial, einem keramischen Material, einem dielektrischen Material und jeglicher Kombinationen daraus besteht.

9. Instrument nach Anspruch 8, bei dem das PTC-Material ein polymer-/kohlenstoffbasiertes Material ist.

10. Instrument nach Anspruch 8, bei dem das PTC-Material ein Cermet-basiertes Material ist.

11. Instrument nach Anspruch 8, bei dem das PTC-Material ein Polymermaterial ist.

12. Instrument nach Anspruch 8, bei dem das PTC-Material ein keramisches Material ist.

13. Instrument nach Anspruch 8, bei dem das PTC-Material ein dielektrisches Material ist.

14. Instrument nach einem der Ansprüche 7 bis 13, bei dem das PTC-Material das an der elektrisch leitfähigen Außenfläche angrenzende Gewebe bei einer Temperatur von unter 100 °C hält.

15. Instrument nach einem der Ansprüche 7 bis 14, bei dem das PTC-Material das an der elektrisch leitfähigen Außenfläche angrenzende Gewebe bei einer Temperatur von über 37 °C hält.

16. Instrument nach einem der Ansprüche 1 bis 15, das eingerichtet ist, eine Ablation basierend auf dem Vergleich der Herzsignale vor der Behandlung und nach der Behandlung durch Abschalten der elektrischen Energiequelle zu beenden.

17. Instrument nach einem der Ansprüche 1 bis 15, bei dem der Herzsensor in dem exponierten Abschnitt bei dem distalen Ende der Elektrode angeordnet ist.

## Revendications

1. Instrument pour l'ablation de tissu, comprenant :
une électrode (11) incluant un élément tubulaire thermoconducteur (15) avec une extrémité distale fermée (12), l'élément tubulaire définissant une surface extérieure électroconductrice externe conçue pour être connectée à une source d'énergie électrique, l'électrode ayant une couche d'isolation (13) disposée sur la surface extérieure électroconductrice externe et une partie exposée à l'extrémité distale ;
au moins un mécanisme de régulation de température pour réguler la température au niveau de la partie exposée de la surface extérieure électroconductrice ; **caractérisé par** :
un capteur cardiaque (102) disposé dans la cavité intérieure de l'électrode configuré pour mesurer les signaux cardiaques avant traitement et après traitement aux fins de comparaison ;
l'appareil comprenant en outre un microprocesseur (44) qui est configuré pour :
comparer les signaux cardiaques avant traitement et après traitement pour déterminer l'évolution de l'ablation tissulaire ; et
terminer l'ablation sur la base de la comparaison des signaux cardiaques avant traitement et après traitement.

2. Instrument selon la revendication 1, dans lequel le capteur cardiaque est disposé de manière adjacente à l'extrémité distale de la surface extérieure électroconductrice.

3. Instrument selon la revendication 1 ou 2, dans lequel l'au moins un mécanisme de régulation de température comprend :
un conduit de fluide (26) défini à l'intérieur de l'élément tubulaire et conçu pour être raccordé à une source (FS) d'alimentation en réfrigérant réglable de manière sélective pour refroidir le tissu contigu à la partie exposée de la surface extérieure électroconductrice.

4. Instrument selon la revendication 3, dans lequel l'au moins un mécanisme de régulation de température comprend en outre :
un capteur de température (23) monté près de la surface extérieure électroconductrice, configuré pour générer un signal de sortie représentatif d'une température près de la surface extérieure électroconductrice, l'alimentation en réfrigérant réglable de manière sélective fournissant du réfrigérant de manière adaptative en fonction de la température près de la surface extérieure électroconductrice.

5. Instrument selon la revendication 4, dans lequel l'alimentation en réfrigérant réglable de manière sélective est configurée pour maintenir automatiquement le tissu contigu à la surface extérieure électroconductrice à une température inférieure à 100°C.

6. Instrument selon la revendication 4 ou 5, dans lequel l'alimentation en réfrigérant réglable de manière sélective est configurée pour maintenir automatiquement le tissu contigu à la surface extérieure électroconductrice à une température supérieure à 37°C.

7. Instrument selon l'une quelconque des revendications précédentes, dans lequel l'au moins un mécanisme de régulation de température comprend un revêtement de matériau (100) à coefficient de température positif (CTP) sur l'électrode.

8. Instrument selon la revendication 7, dans lequel le matériau à CTP est choisi dans le groupe constitué d'un matériau à base de polymère/carbone, d'un matériau à base de cermet, d'un matériau polymère, d'un matériau céramique, d'un matériau diélectrique et de n'importe quelles combinaisons de ceux-ci.

9. Instrument selon la revendication 8, dans lequel le matériau à CTP est un matériau à base de polymère/carbone.

10. Instrument selon la revendication 8, dans lequel le matériau à CTP est un matériau à base de cermet.

11. Instrument selon la revendication 8, dans lequel le matériau à CTP est un matériau polymère.

12. Instrument selon la revendication 8, dans lequel le matériau à CTP est un matériau céramique.

13. Instrument selon la revendication 8, dans lequel le matériau à CTP est un matériau diélectrique.

14. Instrument selon l'une quelconque des revendications 7 à 13, dans lequel le matériau à CTP maintient le tissu contigu à la surface extérieure électroconductrice à une température inférieure à 100°C.

15. Instrument selon l'une quelconque des revendications 7 à 14, dans lequel le matériau à CTP maintient le tissu contigu à la surface extérieure électroconductrice à une température inférieure à 37°C.

16. Instrument selon l'une quelconque des revendications 1 à 15, configuré pour terminer l'ablation sur la base de la comparaison des signaux cardiaques avant traitement et après traitement par coupure de la source d'énergie électrique.

17. Instrument selon l'une quelconque des revendications 1 à 15, dans lequel le capteur cardiaque est disposé à l'intérieur de la partie exposée à l'extrémité distale de l'électrode.
